# EUROPÄISCHE PATENTSCHRIFT

(11) **EP 0 938 379 B1**
(45) Veröffentlichungstag und Bekanntmachung des Hinweises auf die Patenterteilung: **09.10.2002**
(21) Anmeldenummer: 97938844.4
(22) Anmeldetag: 21.07.1997
(51) Int. Cl.: B01J 31/24, C07C 45/50, C07F 9/50

(54) **KATALYSATOR UND VERFAHREN ZUR HERSTELLUNG VON ALDEHYDEN DURCH HYDROFORMYLIERUNG VON OLEFINISCH UNGESÄTTIGTEN VERBINDUNGEN**
CATALYST AND PROCESS FOR THE PRODUCTION OF ALDEHYDES BY HYDROFORMYLATION OF OLEFINICALLY UNSATURATED COMPOUNDS
CATALYSEUR ET PROCEDE POUR LA PRODUCTION D'ALDEHYDES PAR HYDROFORMYLATION DE COMPOSES OLEFINIQUEMENT INSATURES

(30) Priorität: 29.07.1996 DE 19630536; 30.04.1997 DE 19718195
(43) Veröffentlichungstag der Anmeldung: 01.09.1999
(73) Patentinhaber: Celanese Chemicals Europe GmbH, 60439 Frankfurt am Main (DE)
(72) Erfinder: BOGDANOVIC, Sandra, D-60322 Frankfurt am Main (DE); FROHNING, Carl-Dieter, D-46485 Wesel (DE); BAHRMANN, Helmut, D-46499 Hamminkeln (DE); BELLER, Matthias, D-85737 Ismaning (DE); HABER, Steffen, D-61462 Königstein (DE); KLEINER, Hans-Jerg, D-61476 Kronberg (DE)
(86) Internationale Anmeldenummer: EP9703926
(87) Internationale Veröffentlichungsnummer: WO98004346

(56) Entgegenhaltungen:
- BONAPLATA E ET AL: "Hydroformylation of octene-1 with a poly(arylene ether triaryl phosphine) rhodium complex" POLYMER, Bd. 36, Nr. 15, Juli 1995, Seite 3035-3039 XP004025687
- CHEMICAL ABSTRACTS, vol. 1986, Columbus, Ohio, US; abstract no. 460677, TIMMER, KLAAS ET AL: "The synthesis of some polyether bridged diphosphines and their reaction with Rh(COD)acac" XP002043598 & INORG. CHIM. ACTA (1985), 100(2), 235-40 CODEN: ICHAA3;ISSN: 0020-1693,
- CHEMICAL ABSTRACTS, vol. 1996, Columbus, Ohio, US; abstract no. 263310, YAN, YUAN YONG ET AL: "Aqueous-phase rhodium hydroformylation of dodecene-1 with surface-active water-soluble phosphine" XP002043597 & CHIN. CHEM. LETT. (1996), 7(4), 377-80 CODEN: CCLEE7,
- PATENT ABSTRACTS OF JAPAN vol. 096, no. 002, 29.Februar 1996 & JP 07 267890 A (KURARAY CO LTD), 17.Oktober 1995,

## Beschreibung

Die Erfindung betrifft einen neuen Katalysator und ein Verfahren zur Hydroformylierung olefinisch ungesättigter Verbindungen, deren Hydroformylierungsprodukte in Wasser nicht oder nur wenig löslich sind, in Gegenwart dieses Katalysators.

Es ist bekannt, durch Umsetzung von Olefinen mit Kohlenmonoxid und Wasserstoff (Hydroformylierung) Aldehyde und Alkohole herzustellen, die ein Kohlenstoffatom mehr als das Ausgangsolefin enthalten. Die Reaktion wird durch Hydridometallcarbonyle, vorzugsweise solcher der Metalle der VIII. Gruppe des Periodensystems, katalysiert. Neben Kobalt, dem klassischen Katalysatormetall, werden seit einigen Jahren zunehmend Katalysatoren auf Basis von Rhodium eingesetzt. Im Gegensatz zu Kobalt gestattet Rhodium, die Reaktion bei niedrigem Druck durchzuführen, darüber hinaus werden bei Einsatz endständiger Olefine bevorzugt geradkettige n-Aldehyde und nur in untergeordnetem Maße iso-Aldehyde gebildet. Schließlich ist auch die Hydrierung der olefinischen Verbindungen zu gesättigten Kohlenwasserstoffen in Gegenwart von Rhodium-Katalysatoren deutlich niedriger als bei Anwendung von Kobalt-Katalysatoren.

Industriell realisiert ist die Hydroformylierung olefinisch ungesättigter Verbindungen unter der katalytischen Wirkung von Rhodium-Phosphin-Komplexverbindungen im wesentlichen in zwei Varianten. Die eine arbeitet in homogener Phase, d.h. Ausgangsolefin, Katalysatorsystem (Rhodiumcarbonyl und organisches Phosphin) und Reaktionsprodukte liegen gemeinsam in Lösung vor. Die Reaktionsprodukte werden aus dem Gemisch destillativ abgetrennt. Die andere Variante ist durch das Vorhandensein einer vom Reaktionsprodukt getrennten wäßrigen Katalysatorphase, die Rhodiumcarbonylkomplexe und ein sulfoniertes oder carboxyliertes organisches Phosphin enthält, charakterisiert. Diese Ausführungsform erlaubt die Isolierung der Hydroformylierungsprodukte ohne Anwendung thermischer Verfahrensschritte, vereinfacht die Rückführung des Katalysators und ergibt einen besonders hohen Anteil unverzweigter Aldehyde bei Einsatz endständiger Olefine.

Beide Prozesse sind in der Literatur vielfach beschrieben, beispielsweise in W.A. Herrmann, C. W. Kohlpaintner, Angew. Chem. 1993, 105, S.1588 ferner in DE-C-26 27 354 und EP-B-0 103 810.

Bei den in der Technik eingeführten Verfahren wird der Rhodium-Katalysator in Form von Hydridorhodiumcarbonylen eingesetzt, die zusätzliche Liganden, insbesondere tertiäre organische Phosphine oder Phosphite, enthalten. Meist liegen die Liganden im Verhältnis zum Metallatom im Überschuß vor, so daß das Katalysatorsystem aus Komplexverbindungen und freiem Ligand besteht. Der Einsatz der beschriebenen Rhodium-Katalysatoren ermöglicht es, die Hydroformylierungsreaktion bei Drücken unter 300 bar durchzuführen.

Die unterschiedliche Reaktionsführung hat u.a. Einfluß auf die Höhe des Umsatzes der Ausgangsstoffe und die Bildung von Nebenprodukten. Im allgemeinen erzielt man mit dem Verfahren im zweiphasigen Reaktionsmilieu im Vergleich zum homogenen (einphasigen) Verfahren bessere Umsätze bei höherer Selektivität. Ein Vorteil der Umsetzung im System mit gesonderter Katalysatorphase ist die problemlose Abtrennung des Katalysators. Sie erfolgt durch einfaches Scheiden von wäßriger und organischer Phase, d.h. ohne Destillation und damit ohne thermische Verfahrensschritte. In einphasigen homogen katalysierten Verfahren muß dagegen das Reaktionsprodukt vom Katalysator abdestilliert oder der Katalysator muß anderweitig vom Rohprodukt abgetrennt werden. Die Destillation ist wegen der thermischen Empfindlichkeit der Reaktionsprodukte häufig mit Ausbeuteverlusten verbunden. Andere Verfahrensvarianten, wie z.B. die Ausfällung oder eine Membranabtrennung des Katalysators sind verfahrenstechnisch aufwendig und daher unvorteilhaft.

Das zweiphasige Hydroformylierungsverfahren hat sich für die Hydroformylierung von Propen und 1-Buten (aus Butengemischen, z.B. Raffinat 2) im technischen Produktionsmaßstab bewährt. Es ist als Ruhrchemie/Rhöne-Poulenc-Verfahren bekannt. Als Katalysatorsystem findet ein Hydridorhodiumcarbonylkomplex Verwendung, der durch den wasserlöslichen Liganden TPPTS (Triphenylphosphintrisulfonat-Natriumsalz) modifiziert und stabilisiert wird.

Der durch die Sulfonatogruppen wasserlösliche TPPTS-Ligand hat die Funktion, den Rhodiumkomplex in der Wasserphase zu solubilisieren und einen Austrag des Rhodiumkomplexes in die organische Phase zu verhindern. Die chemische Reaktion, d.h. die komplexkatalysierte Addition eines Wasserstoff- und eines Kohlenmonoxidmoleküls an die Doppelbindung findet nach heutiger Vorstellung entweder in der wäßrigen Katalysatorphase oder in der Phasengrenzfläche statt. Das gebildete Produkt kann vermittels der Einstellung des Phasengleichgewichts in die organische Phase gehen.

Für die Hydroformylierung von höheren Olefinen, d.h. Olefinen mit C-Zahlen größer als 6 ist das Ruhrchemie/Rhone-Poulenc-Verfahren nicht geeignet, da nur sehr geringe Raumzeitausbeuten erzielt werden. Die bei zunehmender C-Zahl zu beobachtende Reaktionsverlangsamung bei der Zweiphasenhydroformylierung von Olefinen wird im allgemeinen auf die schlechtere Löslichkeit der höheren Olefine in der Wasserphase zurückgeführt. Da das zweiphasige Hydroformylierungsverfahren sowohl den Vorteil relativ milder Reaktionsbedingungen hat als auch eine einfache Trennung der Produkt- von der Katalysatorphase ermöglicht, besteht ein technisches Interesse daran, auch höhere Olefine nach diesem Verfahren zu hydroformylieren.

Die Hydroformylierungsprodukte höherer Olefine dienen meist als Zwischenprodukte für die Herstellung höherer Alkohole und in geringerem Maßstab zur Herstellung von mittel- bis langkettigen Carbonsäuren durch Oxidation oder zur Synthese von Aminen durch reduktive Aminierung von Aldehyden. Darüber hinaus werden geradkettige Aldehyde mit sieben oder mehr Kohlenstoffatomen in der Riechstoffindustrie als solche oder in Form ihrer Acetale für Parfüms oder zur Parfümierung von Seifen eingesetzt. Lineare und verzweigte Alkohole mit 8 bis 12 C-Atomen finden technisch in großem Umfang Verwendung als Weichmacheralkohole, die zumeist in Form ihrer Bisphthalsäureester oder Bismaleinsäureester als Weichmacher für Weich-PVC eingesetzt werden. Weitere Anwendungsgebiete der höheren, vornehmlich linearen Alkohole sind Komponenten für Waschmittel, Lackrohstoffe und Emailrohstoffe (Ullmanns Encyclopädie der Technischen Chemie, 4. Auflage, Bd. 7, Verlag Chemie Weinheim 1974, S. 118 - 141).
Katalysatoren für die Hydroformylierung sind auch aus J. prakt. Chem. 338 (1996), 124-128 bekannt.

In Chin. Chem. Lett. (YAN, Y. Y. et al.)(1996), 7(4), 377-80 wird ein Verfahren zur Hydroformylierung von 1-Dodecen unter Verwendung eines Katalysators, der Rhodium und ein dreifach (durch Ethylenoxidgruppen) substituiertes Triphenyl-phosphanligand enthält, offenbart.

Es bestand die Aufgabe, ein Verfahren zu entwickeln, das es erlaubt, höhermolekulare olefinisch ungesättigte Verbindungen mit möglichst hoher Aktivität und Selektivität zu den entsprechenden Aldehyden zu hydroformylieren. Darüber hinaus sollen Reaktionsprodukt und Katalysatorsystem leicht voneinander getrennt und Edelmetallverluste weitgehend vermieden werden.

Gelöst wird diese Aufgabe durch einen Katalysator, enthaltend Rhodium und eine Verbindung der allgemeinen Formel (I) 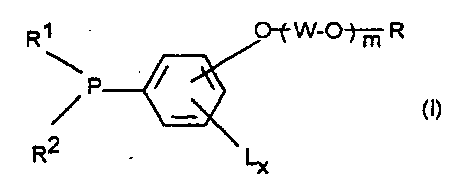 worin
- m: eine Zahl von 1 bis 1000, bevorzugt 2 bis 300, besonders bevorzugt 2 bis 100;
- x: eine Zahl von 0 bis 4, bevorzugt 0 oder 1;
- W: eine Gruppe der Formeln -CH₂-CH₂-, -CH(CH₃)CH₂- oder -CH₂CH(CH₃)-;
- R: Wasserstoff, ein geradkettiger oder verzweigter C₁-C₅-Alkylrest;
oder eine Gruppe der Formeln 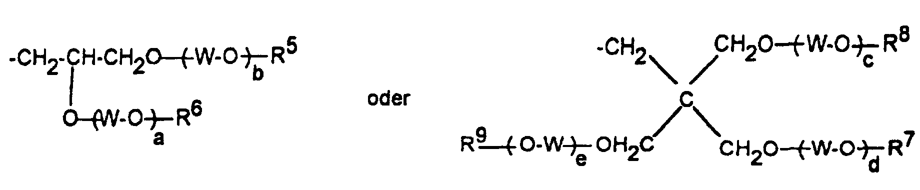 wobei
- a, b, c, d und e: unabhängig voneinander eine Zahl von 0 bis 1000, wobei mindestens eine der Zahlen a, b, c, d und e größer als 0 ist;
- R⁵, R⁶, R⁷, R⁸ und R⁹: gleich oder verschieden sind und Wasserstoff, C₁-C₅-Alkyl oder eine Gruppe der Formel 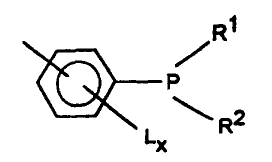 ist,
- R¹ und R²: gleich oder verschieden sind und einen geradkettigen, verzweigten oder cyclischen C₁-C₃₀-Alkylrest oder C₆-C₁₀-Arylrest, der unsubstituiert oder durch ein bis fünf C₁-C₃-Alkylreste substituiert ist, oder R¹ und R² zusammen mit dem dreiwertigen P-Atom ein Dibenzophospholyl der Formel 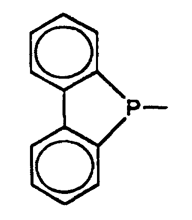 oder ein 3,4-Dimethylphospholyl der Formel 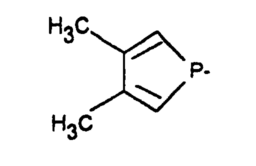 bilden, und
- L: C₁-C₅-Alkyl, C₁-C₅-Alkoxy, NO₂, NR³R⁴, wobei R³ und R⁴ unabhängig voneinander Wasserstoff oder C₁-C₄-Alkyl bedeuten, Cl oder OH bedeuten.

Die Alkylenglykolgruppen am Phenylring können sich in ortho-, meta- oder para-Position zum Phosphoratom befinden. Die der Gruppe -(W-O-)ₘ zugrundeliegende Oxalkylenkette kann ausschließlich aus Ethylenoxid- oder ausschließlich aus Propylenoxid-Einheiten oder aus einer Kombination dieser Einheiten in beliebiger Reihenfolge bestehen.

Von besonderem Interesse sind Verbindungen der Formel (I), worin R¹ und R² gleich sind und jeweils einen geradkettigen oder verzweigten C₁-C₆-Alkylrest, einen Cyclohexylrest oder einen Phenylrest bedeuten.

Von besonderem Interesse sind ferner Verbindungen der Formel (I), worin R Wasserstoff, Methyl, Ethyl, n-Propyl, n-Butyl oder eine Gruppe der Formel 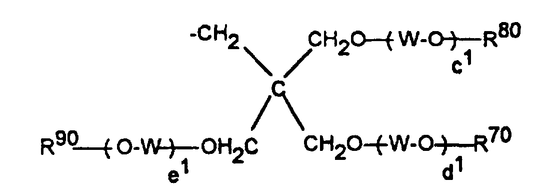 worin c¹, d¹ und e¹ unabhängig voneinander eine Zahl von 1 bis 500, insbesondere 2 bis 300, und
R⁷⁰, R⁸⁰ und R⁹⁰ gleich oder verschieden sind und Wasserstoff, Methyl, Ethyl, n-Propyl oder n-Butyl bedeuten.

Von besonderem Interesse sind ferner Verbindungen der Formel (I), worin L Methoxy, Ethoxy, Methyl, Ethyl oder OH ist oder worin x die Zahl 0 bedeutet.

Beispiele für Verbindungen der Formel (I) sind
Methyl-(triphenylphosphin-4-yl)-tri-ethylenglykol-ether,
Methyl-(triphenylphosphin-3-yl)-tri-ethylenglykol-ether,
Methyl-(triphenylphosphin-2-yl)-tri-ethylenglykol-ether.
sowie Verbindungen mit längeren Oxalkylketten, wobei die Ethoxy- und
Propoxyeinheiten in beliebiger Reihenfolge stehen und in der Regel ein Produktgemisch bilden: 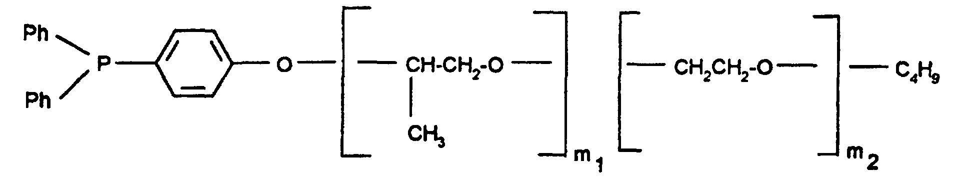 worin m₁ und m₂ jeweils die Zahl 16 und Ph Phenyl bedeuten; 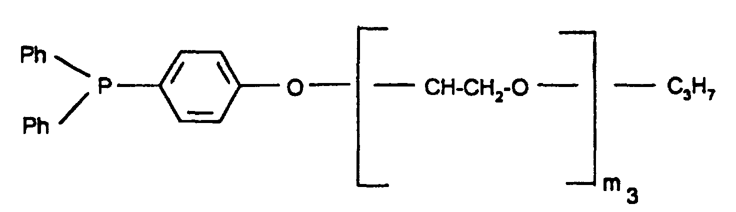 worin m₃ eine Zahl von etwa 22 bedeutet; 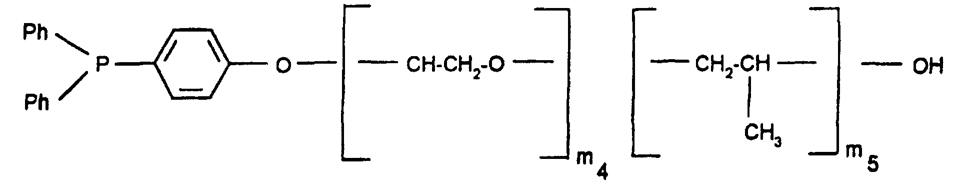 worin m₄ etwa 84 und m₅ etwa 21 bedeutet, 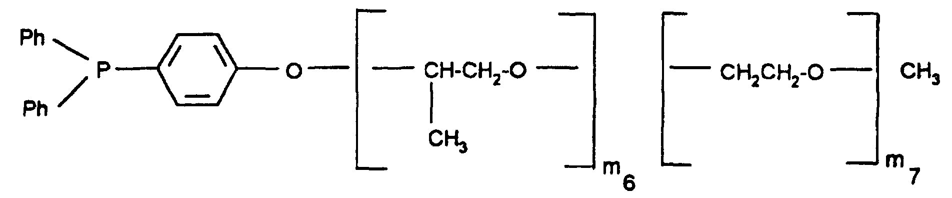 worin m₆ etwa 22 und m₇ 5,5 bedeutet.

Verbindungen der Formel (I) können hergestellt werden, indem man ein Hydroxyphenylphosphan der Formel (II) 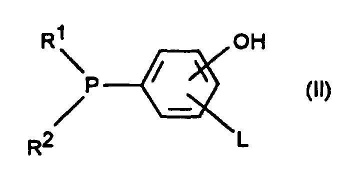 mit einer Base zum entsprechenden Phenolat deprotoniert und mit einer Verbindung der Formel (III)

X-(-W-O-)ₘ-R (III)

worin W, R und m wie vorstehend definiert sind und X eine nucleophil substituierbare Abgangsgruppe ist, zur Verbindung der Formel (I) umsetzt.

Beispiele für die nucleophil substituierbare Abgangsgruppe X sind ortho-, metaoder para-Toluolsulfonat, Methansulfonat, Trifluoracetat, Trifluormethansulfonat, Nonafluorbutylsulfonat, Benzolsulfonat, p-Nitrobenzolsulfonat, Cl oder Br.

Geeignete Basen sind beispielsweise NaOH, KOH, NaH, KH oder Trialkylamine. Bevorzugt ist Triethylamin und KOH.

Die Umsetzung wird zweckmäßigerweise bei Temperaturen zwischen 20 und 100°C, bevorzugt zwischen 60 und 90 °C durchgeführt. Da der Deprotonierungsschritt in der Regel exotherm verläuft, kann zu diesem Zeitpunkt der Synthese eine Kühlung zweckmäßig sein, beispielsweise auf 0 bis 20°C. Das Verfahren kann in Gegenwart oder Abwesenheit organischer Lösungsmittel durchgeführt werden. Als organische Lösungsmittel kommen insbesondere Dimethylformamid, Toluol oder Ethylacetat in Betracht. Es ist weiterhin vorteilhaft, die Umsetzung unter Inertgasatmosphäre durchzuführen.

Der Katalysator läßt sich auf einfache Weise herstellen, indem man Rhodium, beispielsweise in Form eines Salzes oder eines Komplexes mit der Verbindung der Formel (I) zusammenbringt. Beispiele für solche Salze oder Komplexe sind Rhodiumacetat, Rhodiumbutyrat, Rhodiumchlorid, Rhodiumacetylacetonat, Rhodiumnitrat, [RhCl(CO)₂], [Rh(acac)(CO)₂] und HRh(CO)(TPP)₃, wobei acac Acetylacetonat und TPP Triphenylphosphan bedeuten. Besonders günstig ist es, das Rhodium in Form eines wasserlöslichen Salzes oder Komplexes gemeinsam mit einer Verbindung der Formel (I) in Wasser zu lösen. Man kann auch das Rhodiumsalz oder den Rhodiumkomplex zuerst lösen und anschließend die Verbindung der Formel (I) zusetzen oder umgekehrt zunächst die Verbindung der Formel (I) lösen und nachfolgend das Rhodiumsalz oder den Rhodiumkomplex zusetzen.

Man kann den Rhodium und die Verbindung der Formel (I) enthaltenden Katalysator direkt, daß heißt ohne eine zusätzliche Behandlung, in die Hydroformylierung einsetzen.

Es ist allerdings auch möglich, den Rhodium und die Verbindung der Formel (I) enthaltenden Katalysator zunächst einer Vorbehandlung in Gegenwart von Wasserstoff und Kohlenmonoxid unter Druck und gegebenenfalls erhöhter Temperatur zu unterziehen und mittels dieser Präformierung die eigentlich aktive Katalysatorspecies herzustellen. Die Bedingungen für die Präformierung können den Bedingungen einer Hydroformylierung entsprechen.

Der Katalysator enthält üblicherweise Rhodium und die Verbindung der Formel (I) im Molverhältnis 1:1 bis 1:5000, insbesondere 1:100 bis 1:3000. In einer Reihe von Fällen hat sich auch ein Katalysator, der Rhodium und die Verbindung der Formel (I) im Molverhältnis 1:1 bis 1:1:500, insbesondere 1:1 bis 1:200, bevorzugt 1:50 bis 1:150 enthält, als geeignet erwiesen. Im allgemeinen bewirken steigende Mengen an Phosphanliganden eine Verminderung des Verlusts an Edelmetall in die organische Phase bei der Hydroformylierung.

Die vorliegende Erfindung betrifft ferner ein Verfahren zur Herstellung von Aldehyden. Es ist dadurch gekennzeichnet, daß man eine olefinische Verbindung mit 3 bis 20 Kohlenstoffatomen in Gegenwart eines Rhodium und eine Verbindung der allgemeinen Formel (I) enthaltenden Katalysators mit Kohlenmonoxid und Wasserstoff bei einem Druck von 10 bis 500 bar und einer Temperatur von 40 bis 200°C in einem Reaktionsmedium, das aus einer wäßrigen und einer organischen Phase besteht, umsetzt.

Die flüssige organische Phase besteht im wesentlichen aus dem Substratolefin und/oder dem Reaktionsprodukt der Hydroformylierung und gegebenenfalls einem oder mehreren organischen Lösungsmitteln. Wenn ein Lösungsmittel verwendet wird, kann es aus inerten aliphatischen Verbindungen, wie Alkanen, bevorzugt C₅-C₉-Alkane wie Cyclohexan und n-Pentan, oder aromatischen Verbindungen, wie Toluol, Xylol, Ethylbenzol, Mesitylen oder Chlorbenzol, ausgewählt werden.

Die wäßrige Phase enthält den Katalysator und die Verbindung der Formel (I). Es ist vorteilhaft, den Katalysator während der erfindungsgemäßen Hydroformylierung in situ aus vorstehend beschriebenem Rhodiumsalz oder Rhodiumkomplex und der Verbindung der Formel (I) in der wäßrigen Phase zu bilden. Wie vorstehend erwähnt, ist es vorteilhaft, wenn der Katalysator einen stöchiometrischen Überschuß an dem Phosphin der Formel (I) enthält.

Vorteilhafterweise wird daher ein stöchiometrischer Überschuß des Phosphins dem Reaktionsgemisch zugegeben, um den katalytischen Komplex zu bilden und für freies Phosphin zu sorgen. Die freien Phosphine können gleich oder unterschiedlich zu denen sein, die für die Bildung des katalytischen Komplexes verwendet werden, obwohl es vorzuziehen ist, dieselben zu verwenden.

Das Volumenverhältnis von organischer Phase zur wäßrigen Katalysatorphase sollte zwischen 5:1 und 1:5 liegen. Bevorzugt ist ein Bereich von 3:1 bis 1:2. Niedrige Quotienten von wäßriger zu organischer Phase bewirken meist eine Verlangsamung der Reaktionsgeschwindigkeit. Bei hohen Volumenverhältnissen von wäßriger zu organischer Phase ist der Verlust von Rhodium durch Austrag in die organische Phase höher.

Die olefinische Verbindung kann eine oder mehr als eine Kohlenstoff-Kohlenstoff-Doppelbindung enthalten. Die Kohlenstoff-Kohlenstoff-Doppelbindung kann endständig oder innenständig angeordnet sein. Bevorzugt sind olefinische Verbindungen mit endständiger Kohlenstoff-Kohlenstoff-Doppelbindung.

Beispiele für α-olefinische Verbindungen (mit endständiger Kohlenstoff-Kohlenstoff-Doppelbindung) sind Alkene, Alkylalkenoate, Alkylenalkanoate, Alkenylalkylether und Alkenole, insbesondere solche mit 6 bis 14 Kohlenstoffatomen.
Ohne Anspruch auf Vollständigkeit seien als α-olefinische Verbindungen Propylen, 1-Buten, 1-Penten, 1-Hexen, 1-Hepten, 1-Octen, 1-Decen,
1-Dodecen, 1-Octadecen, 2-Äthyl-1-hexen, Styrol, 3-Phenyl-1-propen, Allylchlorid, 1,4-Hexadien, 1,7-Octadien, 3-Cyclohexyl-1-buten, Hex-1-en-4-ol, Oct-1-en-4-ol, Vinylcyclohexen, n-Propyl-7-octenoat, 7-Octensäure, 5-Hexenamid genannt.

Als Beispiele weiterer geeigneter olefinischer Verbindungen seien Buten-2, Diisobutylen, Tripropylen, ®Octol oder ®Dimersol (Dimerisierungsprodukte von Butenen), Tetrapropylen, Cyclohexen, Cyclopenten, Dicyclopentadien, acyclische, cyclische oder bicyclische Terpene, wie Myrcen, Limonen und Pinen erwähnt.

Man setzt den zuvor beschriebenen Rhodium und die Verbindung der Formel (I) enthaltenden Katalysator üblicherweise in einer Menge von 5 bis 100, bevorzugt 30 bis 60, mg Rhodium pro Kilogramm wäßriger Phase ein.

Die Rhodiummenge im Verhältnis zur olefinischen Verbindung beträgt zweckmäßigerweise 1:500 bis 1:100 000, bevorzugt 1:10 000 bis 1:80 000 Mol Rhodium zu Mol olefinischer Verbindung. Daß solche geringen Rhodiummengen für ein zweiphasiges Verfahren ausreichen, ist äußerst überraschend.

Der Rhodiumaustrag in die organische Phase liegt bei dem erfindungsgemäßen Verfahren in den meisten Fällen unter 1ppm.

Der pH-Wert der wäßrigen Phase sollte vorzugsweise bei pH 5 bis 8 liegen. Bei Verwendung eines Puffers sollte dieser mit dem Katalysator verträglich und inert sein.

Man führt die Umsetzung in Gegenwart von Wasserstoff und Kohlenmonoxid (Synthesegas) durch. Das Molverhältnis von Wasserstoff zu Kohlenmonoxid kann in weiten Grenzen gewählt werden und liegt üblicherweise bei 1:10 bis 10:1, insbesondere 5:1 bis 1:5, bevorzugt 2:1 bis 1:2.
Besonders einfach gestaltet sich das Verfahren, wenn man Wasserstoff und Kohlenmonoxid im Molverhältnis 1:1 oder annähernd 1:1 einsetzt.

In einer Vielzahl von Fällen hat es sich als nützlich erweisen, die Umsetzung bei einem Druck von 20 bis 400, insbesondere 30 bis 80 bar durchzuführen. Während 80 bar vom Standpunkt der Aktivität vorzuziehen sind, werden bei 30 bar Synthesegasdruck bessere Selektivitäten in Bezug auf das n/iso-Verhältnis erzielt.

Die Reaktion der olefinischen Verbindungen mit Wasserstoff und Kohlenmonoxid erfolgt bei Temperaturen von 40 bis 200°C. Unterhalb von 40°C wird die Reaktionsgeschwindigkeit unannehmbar langsam, wohingegen eine Katalysatordesaktivierung bei Temperaturen über 200°C auftreten kann. Ein bevorzugter Bereich ist 80 bis 150°C, besonders bevorzugt 110 bis 130°C, da diese Temperaturen die besten Ergebnisse in Bezug auf Selektivität zu Aldehyden ergeben, verbunden mit einer annehmbaren Reaktionsgeschwindigkeit.

Es sei an dieser Stelle darauf hingewiesen, daß die Reaktionsbedingungen, insbesondere Rhodiumkonzentration, Druck und Temperatur auch von der Art der zu hydroformylierenden olefinischen Verbindung abhängen. Vergleichsweise reaktive olefinische Verbindungen erfordern geringe Rhodiumkonzentrationen, niedrige Drücke und niedrige Temperaturen. Hingegen benötigt die Umsetzung relativ reaktionsträger olefinischer Verbindungen größere Rhodiumkonzentrationen, höhere Drücke und höhere Temperaturen.

Das Verfahren läßt sich mit besonders gutem Erfolg ausführen, wenn man eine α-olefinische Verbindung einsetzt. Es lassen sich jedoch auch andere olefinische Verbindungen mit innenständigen Kohlenstoff-Kohlenstoff-Doppelbindungen umsetzen.

Das Hydroformylierungsgemisch wird nach Abschluß einer diskontinuierlichen Umsetzung durch Druckentspannung von Kohlenmonoxid und Wasserstoff befreit und die organische Produktphase von der wäßrigen Katalysatorphase mittels Phasentrennung getrennt. Das erfindungsgemäße Verfahren läßt sich jedoch auch kontinuierlich durchführen.

### Experimenteller Teil

### Herstellung einer Verbindung der Formel (1)

Die Herstellung von Verbindungen der Formel (1) ist in der am gleichen Tag wie die vorliegende Patentanmeldung eingereichten deutschen Patentanmeldung (Aktenzeichen 196 30534.9) beschrieben.

### Herstellung des Katalysators

1) 0,7 mg Rhodium(III)-acetat und 36 g des gemäß Beispiel 4 der vorstehend genannten deutschen Patentanmeldung (Aktenzeichen P 196 30534.9) hergestellten Liganden P-(P41/300)-triphenylphosphan werden entsprechend einem Molverhältnis Rh:Ligand von 1:2500 in 30 ml Wasser gelöst und in einem 200 ml Stahlautoklav bei 25 bar Synthesedruck (CO/H₂ = 1:1) während 3 h auf 125°C gehalten.

### Beispiel 1

### Hydroformylierung von 1-Hexen

In den vorstehend genannten, den Katalysator enthaltenden Stahlautoklaven wurden unter dem bestehenden Druck von 25 bar 30 ml 1-Hexen über eine Pumpe zudosiert und die Mischung 3 Stunden bei 125°C gerührt. Der Synthesegasdruck wurde dabei auf 80 bar erhöht und in einem Druckband von 5 bar konstant gehalten. Nach Reaktionsende wurden Rührer und Heizung abgeschaltet und nach einer Absetzzeit von 30 bis 60 min die obere Produktphase von der Katalysatorphase mittels Phasentrennung getrennt. Die Produktphase wurde mittels Gaschromatographie (GC) und ¹H-NMR-Spektroskopie auf ihren Umsetzungsgrad analysiert:
Umsatz (nach GC) in einer Versuchsreihe von 5 hinterneinander ablaufenden Cyclen mit derselben Katalysatorphase: 97,9 %; 98,4 %, 98,1 %, 97,2 %; 90,4 %. Verhältnis n-Heptanal:iso-Heptanal (nach GC): 71:29

### Beispiele 2 bis 5

Man arbeitete wie in Beispiel 1, änderte jedoch die olefinische Verbindung (jeweils 120 mmol) und den Synthesegasdruck wie in nachfolgender Tabelle angegeben:

| Olefin | Menge Olefin in ml | Synthesegasdruck in bar | Umsatz in % (GC) | Verhältnis n:iso (GC) |
|---|---|---|---|---|
| 1-Octen | 18,9 | 30 | 83,4 | 77:23 |
| 1-Dodecen | 26,8 | 30 | 35,1 | 77:23 |
| 1-Hexen | 15,9 | 80 | 92,8 | 72:28 |
| 1-Octen | 18,9 | 80 | 97,5 | 73:27 |

### Beispiel 6: Versuchsreihe mit 1-Octen (7 Cyclen)

Man arbeitete wie in Beispiel 1, setzte jedoch als Olefin 37,7 ml 1-Octen ein. Man erhielt folgende Umsätze und n:iso-Nonanal-Verhältnisse in den einzelnen Reaktionscyclen:

| Cyclus | Umsatz in % (GC) | Verhältnis n: iso (GC) |
|---|---|---|
| 1 | 97,1 | 71:29 |
| 2 | 97,4 | 71:29 |
| 3 | 97,5 | 71:29 |
| 4 | 97,5 | 70:30 |
| 5 | 97,2 | 71:29 |
| 6 | 95,4 | 70:30 |
| 7 | 97,0 | 69:31 |

### Beispiel 7: Versuchsreihe mit 1-Dodecen (5 Cyclen)

Man arbeitete wie in Beispiel 1, setzte jedoch als Olefin 53,3 ml 1-Dodecen ein. Man erhielt folgende Umsätze und n:iso-Verhältnisse in den einzelnen Reaktionscyclen:

| Cyclus | Umsatz in % (GC) | Verhältnis n:iso (GC) |
|---|---|---|
| 1 | 79,3 | 72:28 |
| 2 | 82,0 | 72:28 |
| 3 | 83,4 | 72:28 |
| 4 | 82,2 | 71:29 |
| 5 | 78,1 | 70:30 |

### Herstellung der in den Beispielen 8 bis 21 verwendeten Katalysatoren

### Allgemeine Herstellungsvorschrift:

6 mg Rhodium(III)acetat und die in der Tabelle I unter "Einwaage Ligand [g]" genannte Menge der gemäß der vorstehend genannten deutschen Patentanmeldung (Aktenzeichen 19 630 534.9) hergestellten Liganden werden entsprechend einem Molverhältnis Rh:Ligand von 1:100 in 30 ml Wasser gelöst und in einem 200 ml Stahlautoklav bei 25 bar Synthesegasdruck (CO/H₂) unter Rühren während 3 Stunden auf 125 °C gehalten (Präformierung). Diese Katalysatorlösung wird in den Beispielen 8 bis 21 eingesetzt.

Bezüglich der verwendeten Verbindungen der Formel(I) (4'-(Diphenylphosphin)-phenoxy-polyalkylenglykole) sei auf die nachfolgende Übersicht verwiesen.

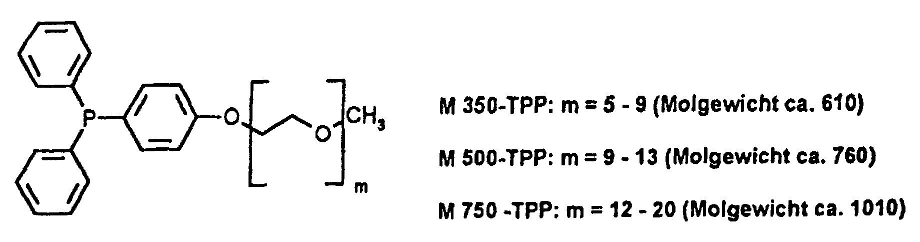

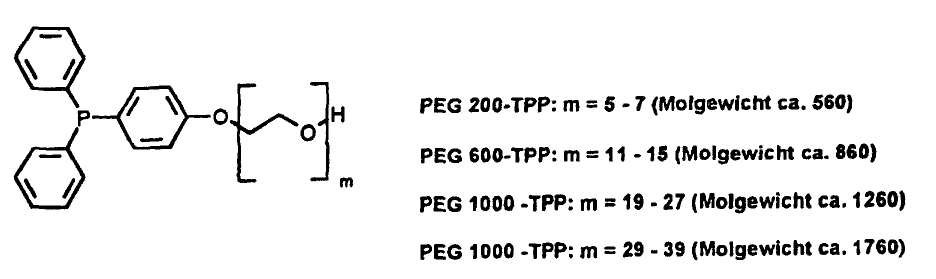

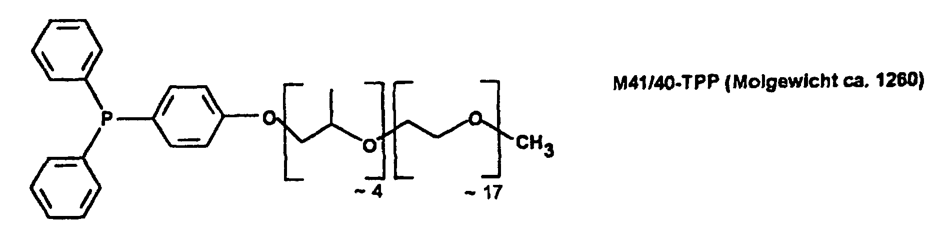

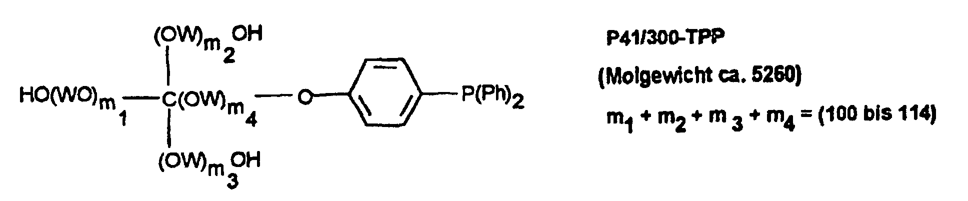

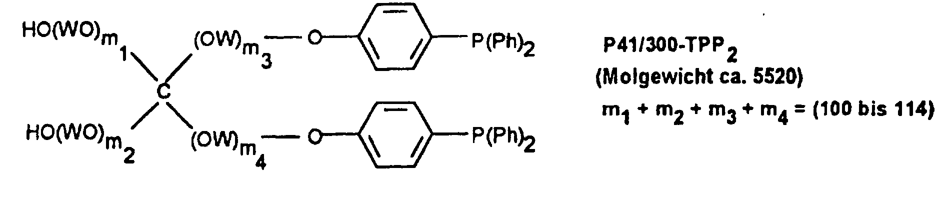

### Beispiel 8: Hydroformylierung von 1-Dodecen mit Rh/M41/40-TPP als Katalysator

In den vorstehend genannten, die Katalysatorlösung enthaltenden Stahlautoklaven werden unter dem bestehenden Druck von 25 bar 26,6 ml 1-Dodecen (120 mmol) über eine Pumpe zudosiert. Der Synthesegasdruck wird sodann auf 50 bar erhöht und in einem Druckband von 5 bar konstant gehalten. Die Reaktionstemperatur beträgt 125 °C. Nach 90 min Reaktionszeit erfolgt keine Gasaufnahme mehr und die Reaktion wird durch Ausschalten des Rührers abgebrochen. Der Autoklav wird auf 25 °C abgekühlt und nach einer Absetzzeit von 60 min wird die obere Produktphase von der Katalysatorphase mittels Phasentrennung getrennt. Die Produktphase wird mittels Gaschromatographie (GC) und ¹H-NMR-Spektroskopie auf ihren Umsetzungsgrad analysiert.

Der Umsatz gemäß GC beträgt 93,4 %.Verhältnis n-Tridecanol : 2-Methyldodecanal ist 72:28.

### Beispiel 9 bis 21: Hydroformylierung von 1-Dodecen mit verschiedenen Katalysatoren enthaltend Rhodium und Verbindungen der Formel (I)

Die folgenden Beispiele werden analog Beispiel 8 bzw. mit kleinen Änderungen der Versuchsparameter sowie unterschiedlichen Verbindungen der Formel (I) durchgeführt. Der Katalysator wird in analoger Weise hergestellt, wobei die in der Tabelle I angegebenen Einwaagen an Ligand verwendet werden. Die weiteren Versuchsparameter sowie die erzielten Versuchsergebnisse sind in der Tabelle I zusammengefaßt.

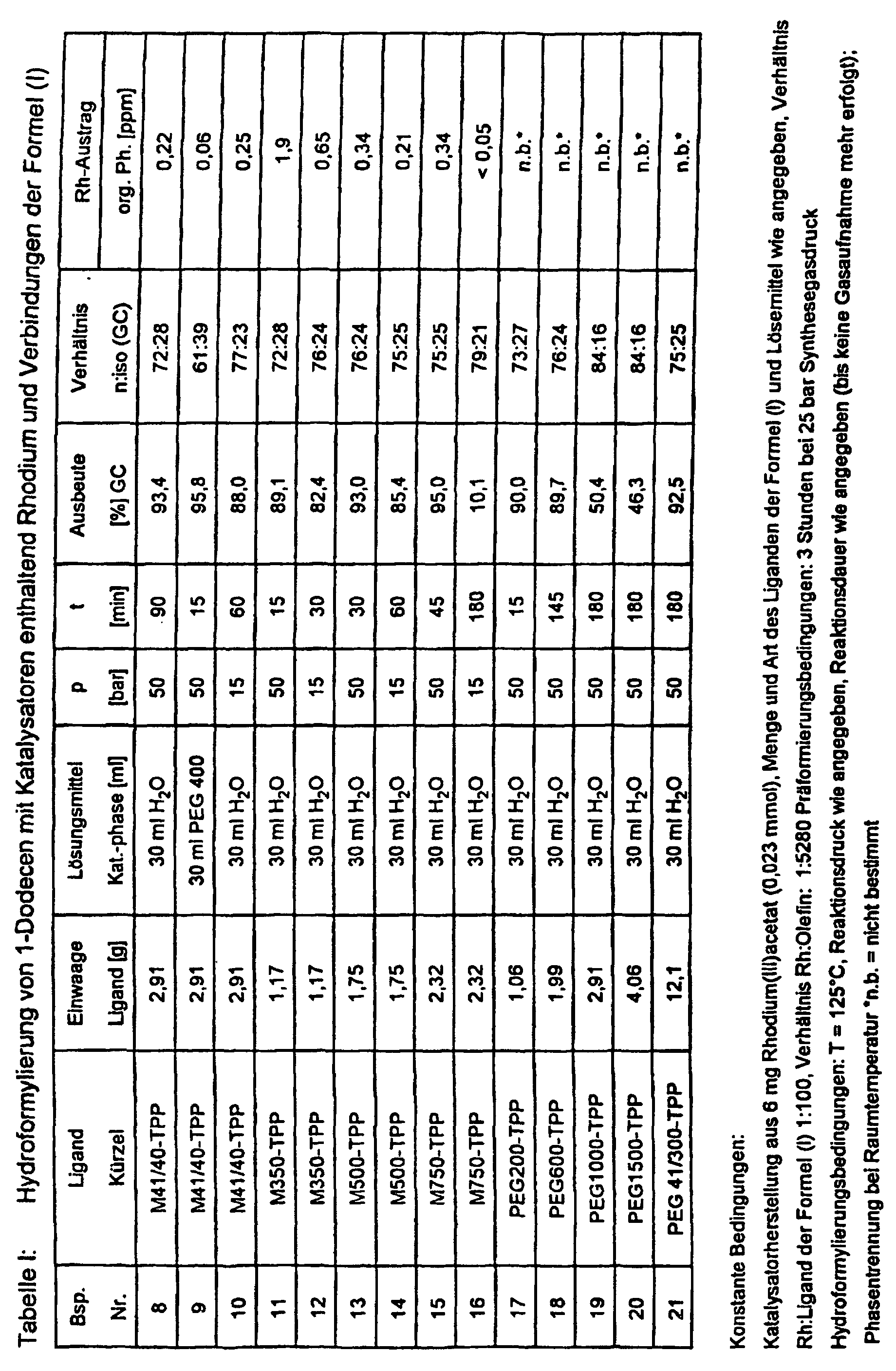

## Patentansprüche

1. Katalysator, enthaltend Rhodium und eine Verbindung der allgemeinen Formel (I) 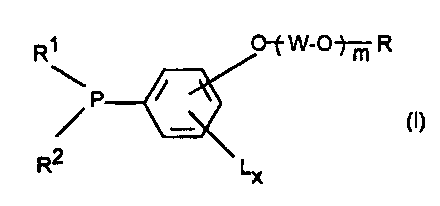 worin
m eine Zahl von 1 bis 1000;
x eine Zahl von 0 bis 4;
W eine Gruppe der Formeln -CH₂-CH₂-, -CH(CH₃)CH₂- oder -CH₂CH(CH₃)-;
R Wasserstoff, ein geradkettiger oder verzweigter C₁-C₅-Alkylrest;
oder eine Gruppe der Formeln 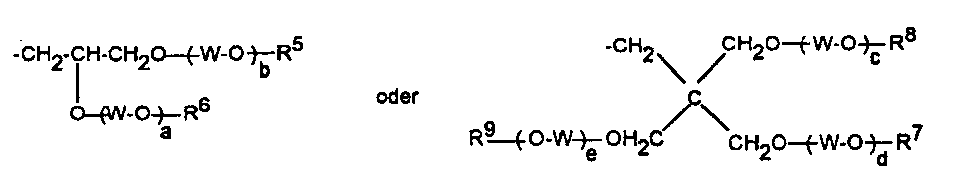 wobei
a, b, c, d und e unabhängig voneinander eine Zahl von 0 bis 1000, wobei mindestens eine der Zahlen a, b, c, d und e größer als 0 ist;
R⁵, R⁶, R⁷, R⁸ und R⁹ gleich oder verschieden sind und Wasserstoff, C₁-C₅-Alkyl oder eine Gruppe der Formel 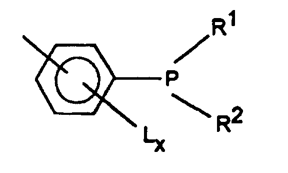 ist,
R¹ und R² gleich oder verschieden sind und einen geradkettigen, verzweigten oder cyclischen C₁-C₃₀-Alkylrest oder C₆-C₁₀-Arylrest, der unsubstituiert oder durch ein bis fünf C₁-C₃-Alkylreste substituiert ist, oder R¹ und R² zusammen mit dem dreiwertigen P-Atom ein Dibenzophospholyl der Formel 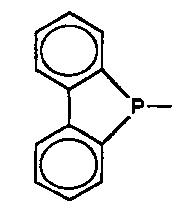 oder ein 3,4-Dimethylphospholyl der Formel 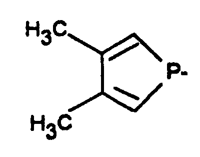 bilden, und
L C₁-C₅-Alkyl, C₁-C₅-Alkoxy, NO₂, NR³R⁴, wobei R³ und R⁴ unabhängig voneinander Wasserstoff oder C₁-C₄-Alkyl bedeuten, Cl oder OH bedeuten.

2. Katalysator nach Anspruch 1, **dadurch gekennzeichnet, daß** R¹ und R² gleich sind und jeweils einen geradkettigen oder verzweigten C₁-C₆-Alkylrest, einen Cyclohexylrest oder einen Phenylrest bedeuten.

3. Katalysator nach Anspruch 1 oder 2, **dadurch gekennzeichnet, daß** R Wasserstoff, Methyl, Ethyl, n-Propyl, n-Butyl oder eine Gruppe der Formel 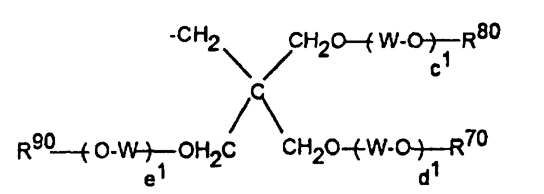 worin c¹, d¹ und e¹ unabhängig voneinander eine Zahl von 1 bis 500, insbesondere 2 bis 300, und
R⁷⁰, R⁸⁰ und R⁹⁰ gleich oder verschieden sind und Wasserstoff, Methyl, Ethyl, n-Propyl oder n-Butyl bedeuten.

4. Katalysator nach mindestens einem der Ansprüche 1 bis 3, **dadurch gekennzeichnet, daß** L Methoxy, Ethoxy, Methyl, Ethyl oder OH ist.

5. Katalysator nach mindestens einem der Ansprüche 1 bis 3, **dadurch gekennzeichnet, daß** x die Zahl 0 bedeutet.

6. Katalysator nach mindestens einem der Ansprüche 1 bis 5, **dadurch gekennzeichnet, daß** er Rhodium und die Verbindung der Formel (I) im Molverhältnis 1:1 bis 1:200 enthält.

7. Verfahren zur Herstellung eines Katalysators nach mindestens einem der Ansprüche 1 bis 6, **dadurch gekennzeichnet, daß** man ein Rhodiumsalz oder ein Rhodiumkomplex mit einer Verbindung der Formel (I) zusammenbringt.

8. Verfahren nach Anspruch 7, **dadurch gekennzeichnet, daß** das Rhodiumsalz oder der Rhodiumkomplex und die Verbindung der Formel (I) in Wasser gelöst werden.

9. Verwendung eines Katalysators nach mindestens einem der Ansprüche 1 bis 6 zur Herstellung von Aldehyden, **dadurch gekennzeichnet, daß** man eine olefinische Verbindung mit 3 bis 20 Kohlenstoffatomen in Gegenwart des Katalysators der allgemeinen Formel (I) mit Kohlenmonoxid und Wasserstoff bei einem Druck von 10 bis 500 bar und einer Temperatur von 40 bis 200°C in einem Reaktionsmedium, das aus einer wäßrigen und einer organischen Phase besteht, umsetzt.

10. Verwendung nach Anspruch 9, **dadurch gekennzeichnet, daß** man eine α-olefinische Verbindung einsetzt.

11. Verwendung nach Anspruch 9 oder 10, **dadurch gekennzeichnet, daß** man den Katalysator entsprechend einer Menge von 10⁻⁵ bis 2x10⁻³ Mol Rhodium pro Mol olefinischer Verbindung einsetzt.

12. Verwendung nach mindestens einem der Ansprüche 9 bis 11, **dadurch gekennzeichnet, daß** mit Kohlenmonoxid und Wasserstoff bei einem Druck von 30 bis 80 bar umgesetzt wird.

13. Verwendung nach mindestens einem der Ansprüche 9 bis 12, **dadurch gekennzeichnet, daß** bei einer Temperatur von 80 bis 150°C umgesetzt wird.

## Claims

1. A catalyst comprising rhodium and a compound of the formula (I) 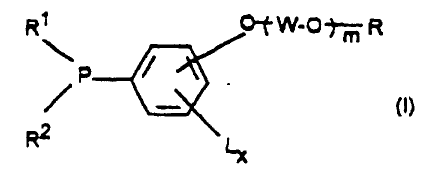 in which
m is a number from 1 to 1000;
x is a number from 0 to 4;
W is a group of the formulae -CH₂-CH₂-, -CH(CH₃)CH₂- or -CH₂CH(CH₃)-;
R is hydrogen, a straight-chain or branched C₁-C₅-alkyl radical;
or a group of the formulae 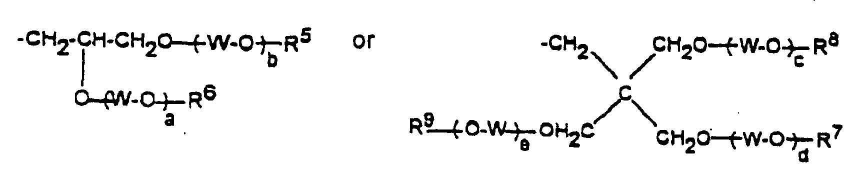 where
a, b, c, d and e independently of one another are a number from 0 to 1000, at least one of the numbers a, b, c, d and e being greater than 0;
R⁵, R⁶, R⁷, R⁸ and R⁹ are identical or different and are hydrogen, C₁-C₅-alkyl or a group of the formula 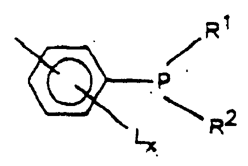
R¹ and R² are identical or different and are a straight-chain, branched or cyclic C₁-C₃₀-alkyl radical or C₆-C₁₀-aryl radical, which is unsubstituted or substituted by from one to five C₁-C₃-alkyl radicals, or R¹ and R² together with the trivalent P atom form a dibenzophospholyl of the formula 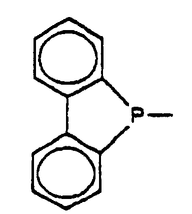 or a 3,4-dimethylphospholyl of the formula 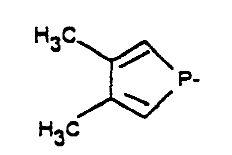 and
L is C₁-C₅-alkyl, C₁-C₅-alkoxy, NO₂, NR³R⁴, where R³ and R⁴ independently of one another are hydrogen or C₁-C₄-alkyl, or L is Cl or OH.

2. The catalyst as claimed in claim 1, wherein R¹ and R² are identical and are each a straight-chain or branched C₁-C₆-alkyl radical, a cyclohexyl radical or a phenyl radical.

3. The catalyst as claimed in claim 1 or 2, wherein R is hydrogen, methyl, ethyl, n-propyl, n-butyl or a group of the formula 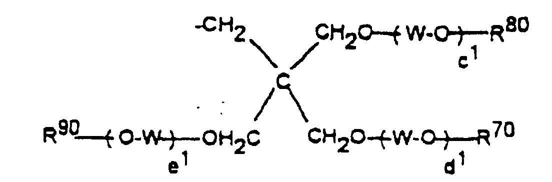 in which c¹, d¹ and e¹ independently of one another are a number from 1 to 500, in particular from 2 to 300, and
R⁷⁰, R⁸⁰ and R⁹⁰ are identical or different and are hydrogen, methyl, ethyl, n-propyl or n-butyl.

4. The catalyst as claimed in at least one of claims 1 to 3, wherein L is methoxy, ethoxy, methyl, ethyl or OH.

5. The catalyst as claimed in at least one of claims 1 to 3, wherein x is 0.

6. The catalyst as claimed in at least one of claims 1 to 5, which comprises rhodium and the compound of the formula (I) in the molar ratio from 1:1 to 1:200.

7. A process for the preparation of a catalyst as claimed in at least one of claims 1 to 6, which comprises bringing together a rhodium salt or a rhodium complex and a compound of the formula (I).

8. The process as claimed in claim 7, wherein the rhodium salt or the rhodium complex and the compound of the formula (I) are dissolved in water.

9. The use of a catalyst as claimed in at least one of claims 1 to 6 for the preparation of aldehydes, which comprises reacting an olefinic compound having from 3 to 20 carbon atoms with carbon monoxide and hydrogen in the presence of the catalyst of the formula (I) at a pressure of from 10 to 500 bar and a temperature of from 40 to 200°C in a reaction medium which comprises an aqueous and an organic phase.

10. The use as claimed in claim 9, wherein an α-olefinic compound is used.

11. The use as claimed in claim 9 or 10, wherein the catalyst is used in an amount corresponding to from 10⁻⁵ to 2x10⁻³ mol of rhodium per mole of olefinic compound.

12. The use as claimed in at least one of claims 9 to 11, wherein the reaction with carbon monoxide and hydrogen is carried out at a pressure of from 30 to 80 bar.

13. The use as claimed in at least one of claims 9 to 12, wherein the reaction is carried out at a temperature of from 80 to 150°C.

## Revendications

1. Catalyseur contenant du rhodium et un composé de formule générale (I) 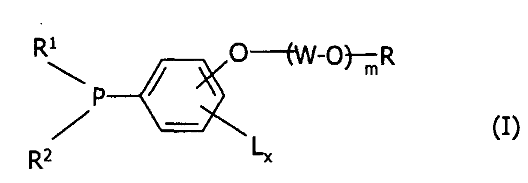 dans laquelle
m est un nombre allant de 1 à 1 000 ;
x est un nombre allant de 0 à 4 ;
W est un groupe de formule -CH₂-CH₂-, -CH(CH₃)CH₂- ou -CH₂CH(CH₃)- ;
R représente l'hydrogène, un groupe alkyle à chaîne droite ou ramifiée en C₁-C₅ ;
ou un groupe de formule 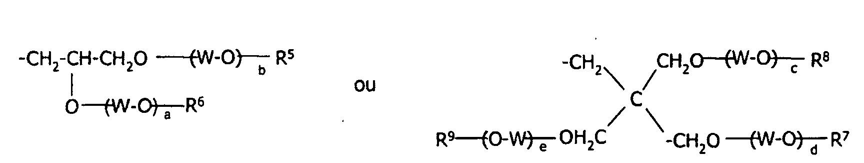 dans lesquelles
a, b, c, d et e sont égaux chacun, indépendamment les uns des autres, à un nombre de 0 à 1 000, l'un au moins des nombres a, b, c, d, et e étant supérieur à 0 ;
R⁵, R⁶, R⁷, R⁸ et R⁹, ayant des significations identiques ou différentes, représentent chacun l'hydrogène, un groupe alkyle en C₁-C₅ ou un groupe de formule 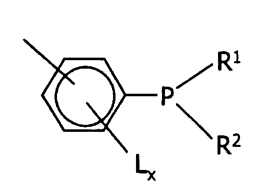
R¹ et R², ayant des significations identiques ou différentes, représentent chacun un groupe alkyle à chaîne droite, ramifiée ou cyclique en C₁-C₃₀ ou un groupe aryle en C₆-C₁₀, non substitué ou portant 1 à 5 substituants alkyle en C₁-C₃, ou bien R¹ et R² forment ensemble et avec l'atome de P trivalent un groupe dibenzophospholyle de formule 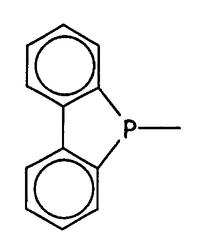 ou un groupe 3,4-diméthylphospholyle de formule 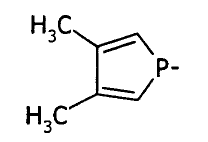 et
L représente un groupe alkyle en C₁-C₅, alcoxy en C₁-C₅, NO₂, NR³R⁴, dans lequel R³ et R⁴ représentent chacun, indépendamment l'un de l'autre, l'hydrogène ou un groupe alkyle en C₁-C₄, Cl ou OH.

2. Catalyseur selon la revendication 1, **caractérisé en ce que** R¹ et R² sont identiques et représentent chacun un groupe alkyle à chaîne droite ou ramifiée en C₁-C₆, un groupe cyclohexyle ou un groupe phényle.

3. Catalyseur selon la revendication 1 ou 2, **caractérisé en ce que** R représente l'hydrogène, un groupe méthyle, éthyle, n-propyle, n-butyle ou un groupe de formule 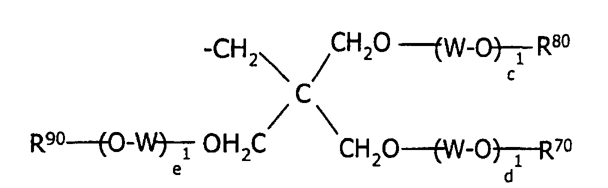 dans laquelle c¹, d¹ et e¹ représentent chacun, indépendamment les uns des autres, un nombre allant de 1 à 500 et plus spécialement de 2 à 300, et
R⁷⁰, R⁸⁰ et R⁹⁰ ayant des significations identiques ou différentes, représentent chacun l'hydrogène, un groupe méthyle, éthyle, n-propyle ou n-butyle.

4. Catalyseur selon au moins une des revendications 1 à 3, **caractérisé en ce que** L représente un groupe méthoxy, éthoxy, méthyle, éthyle ou OH.

5. Catalyseur selon au moins une des revendications 1 à 3, **caractérisé en ce que** x est égal à 0.

6. Catalyseur selon au moins une des revendications 1 à 5, **caractérisé en ce qu'**il contient le rhodium et le composé de formule (I) à un rapport molaire de 1:1 à 1:200.

7. Procédé pour la préparation d'un catalyseur selon au moins une des revendications 1 à 6, **caractérisé en ce que** l'on met en contact un sel ou un complexe de rhodium et un composé de formule (I).

8. Procédé selon la revendication 7, **caractérisé en ce que** l'on dissout le sel ou complexe de rhodium et le composé de formule (I) dans l'eau.

9. Utilisation d'un catalyseur selon au moins une des revendications 1 à 6 pour la préparation d'aldéhydes, **caractérisée en ce que** l'on fait réagir un dérivé oléfinique en C₃-C₂₀ en présence du catalyseur de formule générale (I), avec le monoxyde de carbone et l'hydrogène sous une pression de 10 à 500 bar et à une température de 40 à 200°C dans un milieu de réaction consistant en une phase aqueuse et une phase organique.

10. Utilisation selon la revendication 9, **caractérisée en ce que** le dérivé oléfinique mis en oeuvre est un dérivé α-oléfinique.

11. Utilisation selon la revendication 9 ou 10, **caractérisée en ce que** le catalyseur est mis en oeuvre en quantité correspondant à 10⁻⁵ à 2 x 10⁻³ mol de rhodium par mole du dérivé oléfinique.

12. Utilisation selon au moins une des revendications 9 à 11, **caractérisée en ce que** l'on fait réagir avec le monoxyde de carbone et l'hydrogène sous une pression de 30 à 80 bar.

13. Utilisation selon au moins une des revendications 9 à 12, **caractérisée en ce que** l'on fait réagir à des températures de 80 à 150°C.
